# EUROPEAN PATENT APPLICATION

(11) **EP 1 216 679 A2**
(43) Date of publication of application: **26.06.2002**
(21) Application number: 01850206.2
(22) Date of filing: 07.12.2001
(51) Int. Cl.: A61F 13/72, A61F 13/496, B32B 5/26, D04H 13/00

(54) **Method of producing a laminate of fibrous material, laminate produced according to the method and an absorbent article containing said laminate**

(30) Priority: 20.12.2000 SE 0047621
(71) Applicant: SCA Hygiene Products AB, 405 03 Göteborg (SE)
(72) Inventor: Almberg, Christian, 435 41 Mölnlycke (SE); Kusibojoska, Liljana, 252 49 Helsingborg (SE); Barne, Olle, 426 74 Västra Frölunda (SE); Carlbark, Olle, 417 57 Göteborg (SE); Rönnberg, Peter, 431 33 Mölndal (SE)
(74) Representative: Egeröd, Lisbeth

(57) **Abstract**

Method for producing a laminate of fibrous materials comprising bonding at least one first fibrous material web (1,2) with a first bonding pattern (7), and laminating at least one second fibrous material web (3) to the first material web (1,2) with a second bonding pattern (11), wherein the first and second bonding patterns have bonding areas of different sizes. It is further referred to a laminate produced according to the method an absorbent article provided with a belt, wherein the belt comprises a laminate of the above kind.

## Description

### Technical field

The present invention refers to a method of producing a laminate of fibrous material layers. It also refers to a laminate of fibrous material and an absorbent article containing a laminate of said kind.

### Background of the invention

A plurality of methods are known for producing laminates of fibrous material layers, mainly nonwoven materials of different kind. The different layer can be bonded together by means of heat, at which they are passed through a nip between heated calendar rolls, at least one of which exhibiting a pattern of alternating raised and recessed areas over its envelope surface. The bonding between layers can either be accomplished by at least part of the fibers in the fibrous material are heat meltable, at which the layers are melted together in a bonding pattern corresponding to the pattern of the pattern roll. It is also possible to add a heat activateable bonding agent to at least one layer. An example of a pattern bonded nonwoven material is shown in WO 95/09261.

Another common way of bonding nonwoven materials is by means of ultrasonic bonding, wherein the material webs are fed between an ultrasonic horn and a pattern roll and are bonded in a bonding pattern. A further way is by adhesive bonding wherein the material layers are glued together by means of a bonding agent.

Generally speaking the tighter bonding pattern is used the more stable and stiffer the laminate will be. This occurs partly at the expense of softness and porosity.

Laminates of this kind are used in many different fields. One field is absorbent articles such as diapers, pant diapers, sanitary napkins, incontinence guards and the like, in which nonwoven laminates may be used as breathable backsheet materials, liquid permeable topsheet materials or as a belt to a diaper or incontinence guard for adults, said belt being fastened to or intended to be fastened to the rear and to the front waist part of the article, so that the article assumes a pant-like shape where the belt forms a part of the waist portions of the pant. Examples of belt-provided diapers or incontinence guards are shown in EP-A-0 287 388, EP-A-0 409 307, EP-A-0 528 282, EP-A-0 605 012 and FR-A-2 586 558.

For certain applications it can be desired to combine properties like stability, stiffness, softness against the skin, breathability etc in one and the same laminate. This is for example the case for belts of the above mentioned type, in which the material in the belt must have a certain stability and stiffness for avoiding that the belt forms a "rope" around the waist of the wearer, at the same time as it is soft and comfortable against the skin.

### Object and most important features of the invention

The object of the invention is to provide a method for producing a laminate of fibrous material, where different properties like stiffness, stability, softness etc have been combined in one and the same material. This has according to the invention been provided by bonding at least one first fibrous material web with a first bonding pattern, and laminating at least one second fibrous material web to the first material web with a second bonding pattern, wherein the first and second bonding patterns have bonding areas of different total size.

The invention further refers to a laminate comprising at least one first material layer which is bonded in a first bonding pattern and at least one second fibrous material layer bonded to the first material layer in a second bonding pattern, wherein the first and second bonding patterns have bonding areas of different sizes.

The first fibrous material layer/material web may comprise two or more material layers which are laminated together by the first bonding pattern. Also the second material layer/material web may comprise two or more material layers.

Further embodiments of the invention are disclosed in the dependant claims.

The invention also refers to an absorbent article like diaper or incontinence guard provided with a belt, said belt comprising a laminate of the mentioned kind.

### Description of drawings

The invention will below be closer described with reference to an embodiment shown in the attached drawings.
Fig. 1 shows a schematic side view of a device for performing the method according to the invention.
Fig. 2a and b show examples of different bonding patterns with different bonding areas.
Fig. 3 shows schematically a belt-provided diaper or incontinence guard according to the invention.

### Description of embodiments

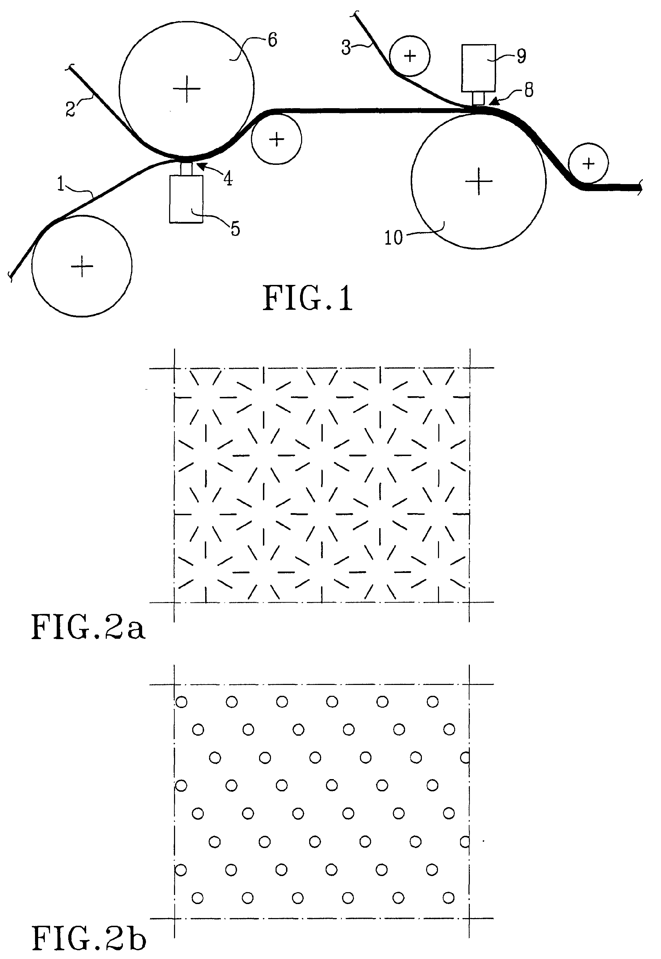

The fibrous material layers that are laminated can be of the same or different type. Preferably they are so called nonwoven materials in which at least a part of the fibers, which may be continuous filaments or staple fibers, are heat meltable fibers. According to the embodiment shown in Fig. 1 two material layers 1 and 2 are fed into a first bonding station 4, which in the embodiment shown is an ultrasonic welding device comprising an ultrasonic horn 5 and a pattern roll 6. The layers 1 and 2 are herewith bonded together in a first bonding pattern 7 having a certain total bonding area or bonding tightness. The two interconnected layers 1 and 2 are then fed to a second bonding station 8 and laminated to a further material layer 3 with a second bonding pattern 11. The bonding station 8 in the embodiment shown is also an ultrasonic welding device comprising an ultrasonic horn 9 and a pattern roll 10. The second bonding pattern, by means of which the third layer 3 is laminated to the layers 1 and 2, is different from the first bonding pattern, by means of which the layers 1 and 2 have been laminated, in such a way that is has a different bonding area than the first bonding pattern.

It is known that the type of bonding pattern and especially the tightness or total bonding area of the bonding pattern effects the properties of the laminate. Generally speaking the larger bonding area the stiffer and more stable laminate is provided. Besides the properties of the laminate are also effected by size and shape of the bonding sites as well as of the configuration with which they are arranged. Large bonding sites like for example lines arranged in a relatively tight pattern thus provide a laminate that is stiffer and more stable than small sparsely arranged bonding sites, for example in the form of points. In the latter case a laminate that is softer and more flexible is achieved.

The term bonding area of a bonding pattern refers to the part of the pattern provided by the bonding sites. The bonding area may vary between about 1% and about 35%. For the tighter bonding pattern the bonding area should be between 4 and 35%, preferably between 5 and 25%. For the more sparse bonding pattern the bonding area should be between 1 and 15%, preferably between 2 and 10%.

The tightness of a bonding pattern, i e the number of bonding sites per unit area, should be between 1 and 25 bonding sites per cm². For the tighter bonding pattern the number of bonding sites should be between 2 and 25 bonding sites/cm², preferably between 4 and 20 bonding sites/ cm². For the more sparse bonding pattern the number of bonding sites should be between 1 and 12 bonding sites/cm², preferably between 2 and 10 bonding sites/ cm². It is pointed out that with relatively large bonding sites, for example in the form of lines, a relatively large bonding area may be provided also with a smaller number of bonding sites as compared to small bonding sites, for example in the form of small points, arranged relatively tighter.

According to the invention one has attempted to combine different properties like stiffness, stability, softness etc in one and the same laminate. By laminating the first and second layers 1 and 2 with a bonding pattern with relatively large bonding area, for example of the kind shown in Fig. 2a, wherein the bonding sites 7a are in the form of relatively short lines arranged in star-like configurations, a certain stiffness of the laminate is provided. The layer which in the bonding station is placed against the pattern roll 6 usually obtains a higher degree of stiffness than the layer placed against the ultrasonic horn 5. It is pointed out that the pattern shown in Fig. 2a only is an example of a bonding pattern which may be used for providing a certain stiffness in the laminate.

The third layer 3 may then be laminated to the first and second layers 1 and 2 with a more sparse bonding pattern 11, for example of the kind shown in Fig. 2b, in which the bonding sites 11a consists of relatively small points. The third layer 3 will then form an outside of the laminate which becomes soft and pliable and for such applications, where the laminate is intended to be used against the body it should be used as the side which is in direct contact with the skin. In order to provide as high degree of softness as possible of the layer 3, this should preferably in the bonding station be placed against the ultrasonic horn 9 and not against the pattern roll 10.

Other alternative ways of laminating the layers together can be by means of heat calendering between two rolls, of which at least one is patterned. It is also possible to combine ultrasonic welding and heat calendering in the same process, so that one lamination step is performed with one method and the other with another method.

It is also pointed out that instead of laminating together two material layers 1, 2 with the first bonding pattern 7 it is possible to bond one material layer with a first bonding pattern 7, said material layer is then laminated to the material layer 3 with the second bonding pattern 11.

At least one and preferably both bonding patterns are preferably non-random patterns consisting of bonding sites in the form of points, lines, spots or the like configured to form patterns.

It has been said above that it is preferred that at least a part of the fibers comprised in the fibrous layers 1-3 are heat meltable. Alternatively it would be possible to add a heat activateable bonding agent to at least one of the layers which are to be bonded together.

Alternative methods for bonding together the layers in the laminate may comprise adhesive bonding. It is also possible to combine adhesive bonding and ultrasonic bonding and/or heat calendaring in the same process, in such a way that two layers are laminated with one method and the third layer with another method. Preferably however the same bonding method is used for all layers.

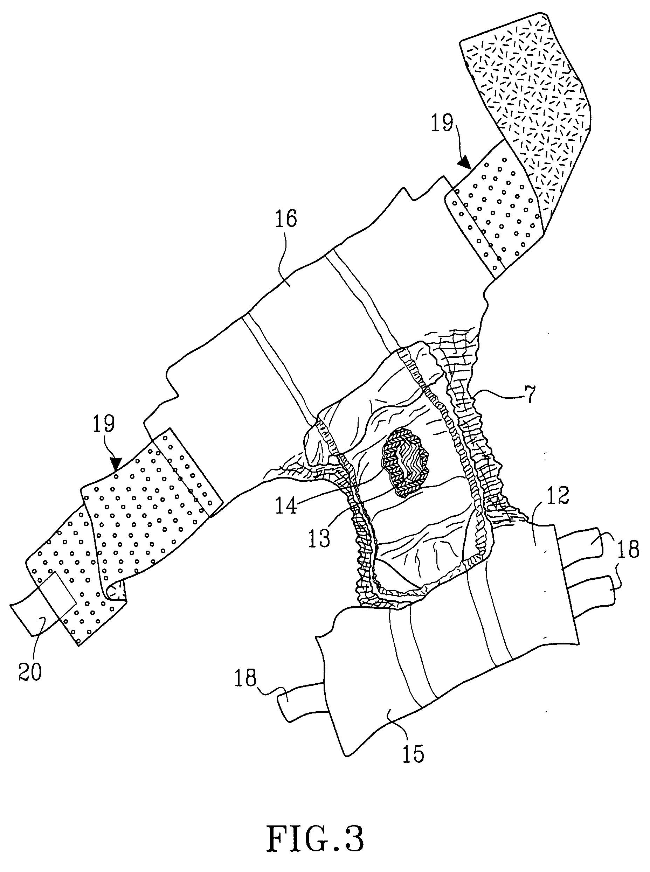

The laminate thus produced can be used in many different fields where it is desired to combine stiffness and stability on one hand and softness on the other hand. Such a field of application is belt in belted diapers and incontinence guards. An example of such a product is shown in Fig. 3. The drawing shows an embodiment of a diaper or incontinence guard 1 comprising a comprising a liquid permeable topsheet 12, a liquid impermeable backsheet 13 and an absorbent body 14 enclosed there between. The liquid permeable topsheet 12 can consist of a nonwoven material, e g a spunbond material of continuous filaments, a meltblown material or a bonded carded fibrous web. The liquid impermeable backsheet 13 may consist of a plastic film, a nonwoven material coated with a liquid impervious material or a hydrophobic nonwoven material which resists liquid penetration.

The topsheet 12 and the backsheet material 13 has a somewhat greater extension in the plane than the absorbent body 14 and extends outside the edges thereof. The layers 12 and 13 are connected to each other within the projecting portions thereof, e g by gluing or welding by heat or ultrasonic.

The absorbent body 14 can be of any conventional kind. Examples of commonly occurring absorbent materials are cellulosic fluff pulp, tissue layers, highly absorbent polymers (so called superabsorbents), absorbent foam materials, absorbent nonwovens or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbents.

The diaper/incontinence guard is intended to enclose the lower part of the wearer's trunk like a pair of absorbent pants. It comprises a front portion 15 intended during use to be worn on the front part of the user's body, a rear portion 16 intended during use to be worn on the rear part of the user's body, and a more narrow crotch portion 17 located between the front and rear portions and which is intended to be worn in the crotch part of the user between the legs. The front portion 15 is provided with a pair of adhesive tape portions 18 or other type of attachment means such as hooks and loops fasteners of the touch-and-close type.

A pair of belt portions 19 are with one end attached, e g glued or ultrasonically welded, to the rear portion 15 of the diaper. The belt portions 19 are with their opposite ends intended to be fastened together, e g by means of tape tab or hook and loop type fastener 20 which is fastened against the outside of the opposite belt portion. The attachment members 18 of the front portion 15 or corresponding attachment means are intended to be attached against the outsides of the belt portions 19 in order to fasten together the diaper/incontinence guard to the desired pantlike shape.

The width of the belt portions 19 should be between 5-20 cm, preferably between 7-15 cm. The belt portions 19 preferably consists of a laminate according to the invention, preferably a laminate of nonwoven materials. The nonwoven materials contained in the different layers may be the same or different. The side of the laminate intended to form the inside of the belt, i e which is intended to be in direct contact with the skin of the wearer, preferably is the layer 3 which is bonded with the more sparse bonding pattern 11 to the two other layers 1 and 2 or to the layer bonded with the tighter bonding pattern 7, in the case this instead of the two laminated layers 1 and 2 consists of one single layer which has been bonded in a bonding pattern 7. The middle layer, i e the layer 2, preferably is the layer that provides stiffness and stability to the laminate, and should therefor be the material that during the lamination process is located closest to the pattern roll 6. The layer 3, which should form the inside of the belt, should however preferably not be located closest to the pattern roll 10 during the lamination process. Instead it would be appropriate that the layer 1, which is intended to form the outside of the belt and thus serve as a receiving surface for the fastening means 18 and 20 respectively, during the lamination process, is the layer that is located closest to the pattern roll 10. By this the layer 1 is given a certain three-dimensional structure, which is an advantage for its function as a receiving surface for the fastening means 18 and is an advantage for its function as a receiving surface for the fastening means 18 and 20, especially if these are hook members to a hook-and-loop type fastening means, at which the layer 1 forms the loop members of the hook-and-loop type fastener, with which the hook members cooperate.

It is important that the material which is intended to be located closest to the skin is soft and bulky and has a low surface friction. A suitable material can be a carded so called through-air-bonded nonwoven material.

The invention is of course not limited to the embodiments described above and shown in the drawings but can be varied within the scope of the claims. Thus the bonding patterns can be varied as well as type of bonding method, as long as the intended difference in bonding patterns between the different layers is provided. It is also possible to laminate together more than three layers with the method according to the intention, at which at least two different bonding patterns are used in the different bonding stations.

## Claims

1. Method for producing a laminate of fibrous materials,
**characterized in**
bonding at least one first fibrous material web (1,2) with a first bonding pattern (7), and laminating at least one second fibrous material web (3) to the first material web (1,2) with a second bonding pattern (11), wherein the first and second bonding patterns have bonding areas of different sizes.

2. Method as claimed in claim 1,
**characterized in**
**that** at least one bonding pattern (7,11) is provided by means of heat or ultrasonic.

3. Method as claimed in claim 1 or 2,
**characterized in**
**that** at least one bonding pattern (7,11) is a non-random bonding pattern consisting of bonding sites in the form of points, lines, spots or the like.

4. Method as claimed in any of the preceding claims,
**characterized in**
**that** the first material web (1,2) is bonded over a first pattern roll (6), which is intended to provide the first bonding pattern (7), which has the larger bonding area, at which the side (2) of the first material web which is intended to form the inside of the produced laminate is located closest to the pattern roll (6) during the lamination process.

5. Method as claimed in any of the preceding claims,
**characterized in**
**that** the second material web (3) is laminated to the first material web (1,2) over a second pattern roll (10), which is intended to provide the second bonding pattern (11), which has the smaller bonding area, at which the first material web (1), which is intended to form one of the external sides of the laminate, is located closest to the second pattern roll (10) during the lamination process.

6. Method according to claim 1 or 2,
**characterized in**
**that** at least one bonding pattern (7,11) is provided by adhesive bonding.

7. Method as claimed in any of the preceding claims,
**characterized in**
**that** the first material web comprises at least two fibrous material layers (1,2) which are laminated together by the first bonding pattern (7).

8. Method as claimed in any of the preceding claims,
**characterized in**
**that** the second material web (3) comprises at least two fibrous material layers.

9. Laminate comprising fibrous materials,
**characterized in**
at least one first material layer (1,2) which is bonded in a first bonding pattern (7) and
at least one second fibrous material layer (3) bonded to the first material layer in a second bonding pattern (11), wherein the first and second bonding patterns have bonding areas of different sizes.

10. Laminate according to claim 9,
**characterized in**
**that** at least one bonding pattern (7,11) is provided by means of heat or ultrasonic.

11. Laminate according to claims 9 or 10,
**characterized in**
**that** at least one of the bonding patterns (7,11) is a non-random pattern comprising bonding sites in the form of points, lines, spots or the like.

12. Laminate according to any of claims 9-11,
**characterized in**
**that** at least one of the bonding patterns (7,11) is provided by adhesive bonding.

13. Laminate according to any of claims 9-12,
**characterized in**
**that** the first material layer comprises at least two fibrous material layers (1,2) which are laminated together by the first bonding pattern (7).

14. Laminate according to any of claims 9-13,
**characterized in**
**that** the second material layer (3) comprises at least two fibrous material layers.

15. Laminate according to any of claims 9-14,
**characterized in**
**that** the first bonding pattern (7) has a bonding surface between 4 and 35%, preferably between 5 and 25.

16. Laminate according to any of claims 9-15,
**characterized in**
**that** the first bonding pattern (7) has between 2 and 25 bonding sites per cm² and preferably between 4 and 20 bonding sites per cm².

17. Laminate according to any of claims 9-16,
**characterized in**
**that** the second bonding pattern (11) has a total bonding area between 1 and 15%, preferably between 2 and 10%.

18. Laminate according to any of claims 9-17,
**characterized in**
**that** the second bonding pattern (11) has between 1 and 12 bonding areas per cm² and preferably between 2 and 10 bonding sites per cm².

19. Absorbent article such as a diaper and an incontinence guard comprising a liquid permeable topsheet (12), a liquid impermeable backsheet (13) and an absorbent body (14) enclosed there between, said article having a front portion (15), a rear portion (16) and a crotch portion (17) there between, and further is provided with a belt (19) attached or intended to be attached to the rear portion (16) and to the front portion (15) of the article in such a way that the article will assume a pantlike shape, where the belt (19) forms a part of the waist portions of the pant,
**characterized in**
**that** the belt (19) comprises a laminate of the kind stated in any of claims 1-18.
